(19) Europäisches Patentamt

European Patent Office

Office européen des brevets

(11) **EP 1 186 059 B1**

(12) **EUROPEAN PATENT SPECIFICATION**

(45) Date of publication and mention
of the grant of the patent:
**01.03.2006 Bulletin 2006/09**

(21) Application number: **00928141.1**

(22) Date of filing: **06.04.2000**

(51) Int Cl.:
*H01L 37/02* (2006.01)    *H01L 41/18* (2006.01)
*C12N 11/02* (2006.01)    *C12N 11/08* (2006.01)
*G01N 33/544* (2006.01)    *G01N 33/545* (2006.01)
*B32B 9/00* (2006.01)    *B32B 9/04* (2006.01)
*C08F 20/54* (2006.01)    *C08F 20/58* (2006.01)
*C08F 20/60* (2006.01)    *B05D 1/20* (2006.01)
*C07H 15/04* (2006.01)    *C07H 15/00* (2006.01)
*C07H 23/00* (2006.01)

(86) International application number:
**PCT/US2000/009216**

(87) International publication number:
**WO 2000/062351 (19.10.2000 Gazette 2000/42)**

(54) **Method for forming monolayers**

Verfahren zur Herstellung von Monoschichten

Procédé de formation de monocouches

(84) Designated Contracting States:
**AT BE CH CY DE DK ES FI FR GB GR IE IT LI LU
MC NL PT SE**

(30) Priority: **09.04.1999 US 128591 P
02.12.1999 US 452968**

(43) Date of publication of application:
**13.03.2002 Bulletin 2002/11**

(73) Proprietor: **Auburn University
Auburn, AL 36849-5176 (US)**

(72) Inventors:
• **VODYANOY, Vitaly Jacob
Auburn, AL 36830 (US)**
• **CHIN, Bryan Allen
Auburn, AL 36830 (US)**
• **BARBAREE, James Martin
Dadeville, AL 36853 (US)**
• **NEELY, William Charles
Auburn, AL 36830 (US)**
• **PATHIRANA, Suram Therese
Sunnyvale, CA 94086 (US)**
• **HSIEN, Yun-Hwa Peggy
Auburn, AL 36830 (US)**
• **ZEE, Ralph Hing-Chung
Auburn, AL 36830 (US)**
• **WIKLE, Howard Clyde, III
Auburn, AL 36830 (US)**

(74) Representative: **Bentham, Andrew
J.A. Kemp & Co.
14 South Square
Gray's Inn
London WC1R 5JJ (GB)**

(56) References cited:
**WO-A-94/18572          WO-A-98/27417
WO-A1-87/00347          US-A- 4 554 076
US-A- 5 019 451          US-A- 5 102 798
US-A- 5 120 809          US-A- 5 510 481
US-A- 5 580 612**

• **A. BARRAUD ET AL: "Study of immunoglobulin
G thin layer obtained by the Langmuir-Blodgett
method: applications to immunosensors"
BIOSENSORS BIOELECTRONICS, vol. 8, 1993,
pages 39-48, XP002306419**
• **CHUDINOVA G K ET AL: "The study of the
antigen-antibody reaction by fluorescence
method in LB films for immunosensor" THIN
SOLID FILMS, ELSEVIER-SEQUOIA S.A.
LAUSANNE, CH, vol. 307, no. 1-2, 10 October 1997
(1997-10-10), pages 294-297, XP004109354 ISSN:
0040-6090**
• **C. NICOLINI ET AL: "Quartz balance DNA sensor"
BIOSENSORS BIOELECTRONICS, vol. 12, no. 7,
1997, pages 613-618, XP002306420**

EP 1 186 059 B1

- **VIKHOLM I ET AL: "In situ quartz crystal microbalance monitoring of Fab'-fragment binding to linker lipids in a phosphatidylcholine monolayer matrix. Application to immunosensors" PREPARATION AND CHARACTERIZATION, ELSEVIER SEQUOIA, NL, vol. 327-329, 31 August 1998 (1998-08-31), pages 643-646, XP004355793 ISSN: 0040-6090**

- **SMITH ET AL.: 'Optimization of thermal performance of langmuir-blodgett film pyroelectric devices' THIN SOLID FILMS, vol. 146, 1987, pages 7 - 13, XP002929979**
- **SMITH ET AL.: 'Pyroelectric activity in non-centrosymmetric langmuir-blodgett multilayer films' THIN SOLID FILMS, vol. 132, 1985, pages 125 - 134, XP002929980**

**Description**

**FIELD OF THE INVENTION**

The invention relates to methods for forming monolayers, and in particular to monolayers containing antibodies and uses thereof.

**BACKGROUND OF THE INVENTION**

A monolayer is a one molecule thick film of at least one amphiphilic compound or composition that forms at the air/water interface of an aqueous solution. Each molecule in the monolayer is aligned in the same orientation, with the hydrophobic domain facing the air and the hydrophilic domain facing the aqueous solution. Compression of the monolayer results in the formation of an ordered two dimensional solid that may be transferred to a substrate by passing the substrate through the monolayer at the air/water interface. A monolayer that has been transferred to a substrate is termed a Langmuir-Blodgett film, or LB film. For reviews of Langmuir-Blodgett technology, see Gaines, G.L. Jr. (1965). *Insoluble Monolayers at Liquid-Gas Interfaces.* Interscience, New York; Zasadzinski *et al.* (1994) *Science 263*: 1726-1733; Ullman (1991) *An Introduction to Ultrathin Organic Films,* Academic Press, Boston, MA; and Roberts (1990) *Langmuir-Blodgett Films,* Plenum, New York.

Monolayers are typically composed of organic molecules such as lipids, fatty acids and fatty acid derivatives, fat soluble vitamins, cholesterol, chlorophyll, valinomycin and synthetic polymers such as polyvinyl acetate and polymethyl methacrylate, but may also be formed by many other amphiphilic compounds. LB films may be used to detect a molecule that binds to or reacts with a compound of interest that comprises the monolayer or has been incorporated into the monolayer.

Sensing systems employing LB films include electrochemical devices using ion-sensitive field effect transistors, absorption or fluorescence based optical devices, and piezoelectric crystals. For example, LB films of valinomycin have been used to detect a specific interaction of potassium that results in a conformational change that is detectable by infrared spectroscopy (Pathairana *et al.* (1992) *Langmuir 8*:1984-1987).

Monolayers incorporating fluorescein lipids have been deposited on quartz crystal microbalances and used to detect specific anti-fluorescyl monoclonal antibodies in solution (Ebato *et al.* (1994) *Analytical Chem.* 66:1683-1689). In contrast, detection of antigens by piezoelectric crystals coated with LB films incorporating antibodies has met with limited success. In these systems, non-specific binding of molecules to the LB film prevents accurate measurement of antigen.

Previous methods for forming LB films require dissolution of the compounds to be formed into a monolayer in a volatile organic solvent. The organic solvent forms a separate phase from the aqueous solution and functions to prevent dissolution of the monolayer components in the aqueous phase. After spreading the mixture at the air-liquid interface of the aqueous solution, the solvent is allowed to evaporate, leaving a monolayer at the interface. Unfortunately, the organic solvent often damages the monolayer components and leaves an undesirable residue. LB films formed from such monolayers may have unacceptable levels of nonspecific binding. Such non-specific binding, which is non-saturable, hampers quantitative measurement of specific binding. The present invention overcomes these problems by providing a method for forming monolayers that does not require the use of an organic solvent.

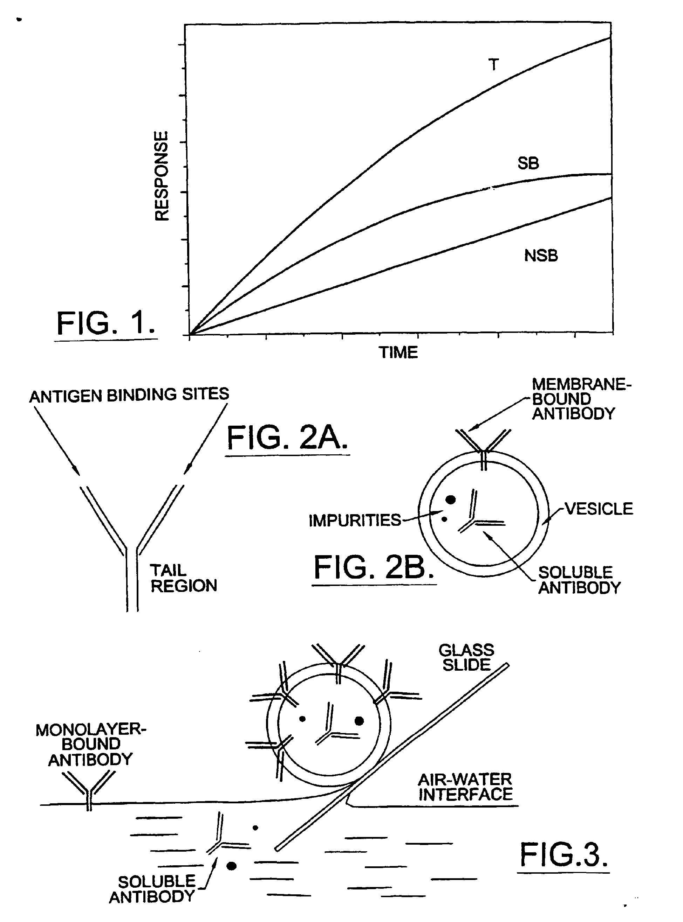

The efficient sensing interaction in the immunosensor requires (1) high surface density of functional molecules, (2) high specificity of interactions and the absence of non-specific binding, (3) accessibility of interacting partners, and (4) stability of the sensing system. There is one important feature of any immunosensor, that from the practical point of view is the most important. This feature is a dynamic response-time curve of the sensor. If an immunosensor is exposed to a specific antigen, the dynamic output signal Vs time represents the binding process, which usually looks like curve T shown in Figure 1. The total binding (T) includes a component of non-specific binding (NSB), which is non-saturable, and the reminder is specific binding (SB) which saturates, when interaction of antigen-antibody (Ag-Ab) reaches a steady state level. The ability of the Ag-Ab system to achieve a steady state level is extremely important, because only this level of specific binding is a direct measure of the target antigen concentration in the analyte. Unfortunately, extra antigens are by damaged antibodies or by other binding sites. In this case the sensor output corresponding to a steady-state level of the specific binding is masked for this case by the continuously growing contribution of non-specific binding. In practice, to relate concentrations of target antigens in analyte to sensor output, volume of liquid, flow rate of liquid or time of exposure, must be controlled and/or known. In contrast, when non-specific binding is low and steady state is achieved, each steady state level corresponds to a specific concentration of antigen. Thus, for the best results, surface density and specificity of antibodies must be high and have the same affinity, and non-specific bindings have to be small.

Many problems of antibody immobilization originate from the very nature of the antibody itself. The typical antibodies are Y-shared molecules (2 Fab plus Fc immunoglobulin structure with two potential antigen binding sites located on the variable region of the Fab fragments (Figure 2A). All classes of antibody produced by B lymphocytes can be made in a membrane-bound form and in soluble secreted form. The two forms differ only in their carboxyl terminals: the membrane-bound form has a hydrophobic tail (Fc) which anchors it in the lipid bilayer of B cell membrane, whereas the secreted form has a hydrophilic tail, which allows them to escape from the cell (Figure 2B). Only one of these two forms, the form with a hydrophobic tail, can be held by monolayers, is uniquely qualified to be used in the Langmuir-Blodgett techniques. This form is suitable for proper alignment and orientation in sensor membranes (Figure 3).

Antibodies derived from immunized animals in the form of antisera or purified protein preparations may contain different impurities and antibodies in both the membrane-bound and soluble form. Previous methods for forming LB monolayers that use organic solvents as a spreading carrier drag these impurities and both forms of antibodies into the monolayer. These methods may produce monolayers with high densities of antibodies but residuals of organic solvent, impurities, and entrapped hydrophilic antibodies that may destabilize the monolayer and inhibit the antibody-antigen interactions. A comparative study of antibody immobilization techniques for immunosensors concluded that the conventional LB technique produced the highest density of coating, but with very high non-specific binding and poor reproducibility, indicating that the film did not have a well organised structure (Ahluwalia *et al.* (1991) *Biosensors and*

*Bioelectronics* 7:207-214).

Additionally, US-A-5,102,798 provides Langmuir-Blodgett films having hydropbilic surfaces for immobilization of active moieties having bioactive, immunoactive, thermoactive, electroactive, optactive or redoxactive properties.

Also, US-A-5,120,809 provides amphiphilic monomers with mixed-chain structures of a defined formula. The momomers are polymerized on their own or together with other comonomers. The polymers obtained are suitable for the preparation of ultra-thin layers on a suitable layer support.

Also, in Chudinova *et al.* (1997) *(Thin Solid Films 307,294-297),* the antigen-antibody reaction was investigated for immunosensors in LB lipid monolayers by a fluorescence method. Barraud *et al. (Biosensors & Bioelectronics 8 (1993), 39-48)* have also investigated immunoglobulin G thin layers obtained by the Langmuir-Blodgett method and their application to immunosensors and Nicolini *et al. (Biosensors & Bioelectronics Vol. 12 No. 7, pp 613-618, 1997)* have investigated the applicabilty of the Langmuir-Blodgett technique to the development of quartz balance DNA sensors.

Further, Vikholm *et al. (Thin Solid Films 327-329 (1998), 643-646)* have studied *in situ* quartz crystal microbalance monitoring of Fab-fragment binding to linker lipids in a phosphatidylcholine monolayer matrix.

Also, WO 98/27417 provides biosensing devices which produce diffraction images. The devices of WO 98/27417 are intended for detecting and quantifying analytes present in a medium. The device comprises a metalized film upon which is printed a specific, predetermined pattern of a analyte-specific receptors. Upon attachment of a target analyte to select areas of the plastic film upon which the receptor is printed, diffraction of transmitted and/or reflected light occurs via the physical dimensions and defined, precise placement of the analyte. A diffraction image is produced which can be easily seen with the dye or, optionally, with a sensing device.

Finally, WO 94/18572 provides a method for the detection of ABO and RH systems in red blood corpuscles wherein is used a microtiter strip where monolayers of positive red blood corpuscles have been previously formed and sensitized with antiserum.

The present invention overcomes these problems by providing the method for forming monolayers containing antibodies with high specificity and low non-specific binding. As a result, a dynamic equilibrium (association-dissociation) of antibody-antigen interaction can be rapidly achieved and measured.

## SUMMARY OF THE INVENTION

Methods for forming monolayers without the aid of an organic solvent are provided. In particular, methods for the formation of monolayers from serum or antibody-coupled liposomes are provided. Also provided are monolayers and Langmuir Blodgett films formed by the methods of the invention and comprising at least one amphiphilic compound and optionally, one or more compounds of interest, are provided. Additionally provided are substrates, such as piezoelectric crystals, having deposited thereon the films of the invention. The monolayers, films and substrates of the invention are useful as components of biosensors and/or chemosensors and in the study of surface chemistry, biology, environmental science and medical applications.

Accordingly, the invention provides a method for forming a monolayer, comprising:

(a) providing a composition comprising at least one amphiphilic compound, wherein said composition contains not more than 25% of a volatile organic solvent and may optionally contain one or more compounds of interest;
(b) immersing one end of a wettable planar surface into an aqueous subphase, wherein said planar surface forms an angle of about 90-170 degrees to an upper surface of said subphase, and said subphase comprises at least one monovalent cation and at least one bivalent cation;
(c) delivering said composition at a rate of about 0.02-4.0 ml per minute to said planar surface to form a monolayer; and

(d) compressing said monolayer to a desired surface pressure.

The invention also provides a method as described immediately above, further comprising depositing at least one such monolayer on a substrate to form an LB film.

BRIEF DESCRIPTION OF THE DRAWINGS

Figure 1. Graphical representation of a binding process with specific and non-specific components.
Figure 2. Schematic representation of antibodies (Panel A) and a lipid vesicle with membrane-bound antibody, soluble antibody and impurities inside the vesicle (Panel B).
Figure 3. Schematic representation of a monolayer forming from a vesicle containing membrane bound and soluble antibodies.

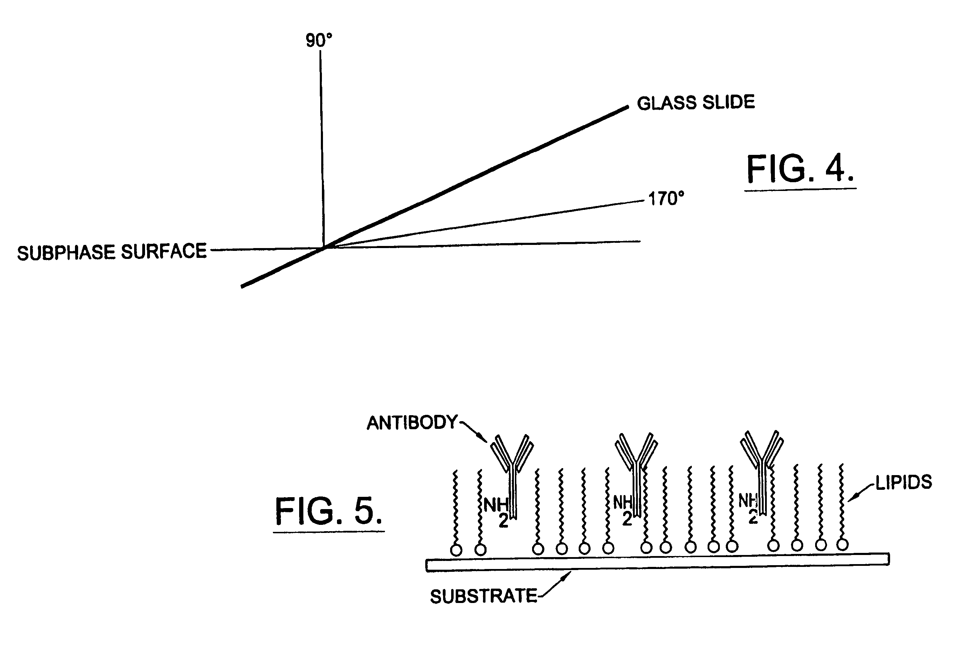

Figure 4. Schematic representation of the glass slide during spreading of solutions containing antibodies.
Figure 5. Schematic representation of antibodies immobilized in the lipid monolayer.
Figure 6. *Salmonella* detection.

Panel A. A graphical representation of the voltage with respect to time for *Salmonella* sensor in response to various bacterial concentrations. Seven monolayers of the *Salmonella* O Antibody Serum were transferred to a sensor quartz crystal substrate using the Langmuir-Blodgett technique. These films were made at 19°C on a subphase containing 55mM KCl, 4mM NaCl, 1mM $MgCl_2$, 0.1 mM $CaCl_2$ and 2mM MOPS with a pH of 7.4. The film covered crystal was placed into the probe cavity of the MAXTEK microbalance and 1 ml of the following solutions were applied in sequence, and the change in voltage was recorded for 8 minutes for each solution at 25°C. Subphase solution and *Salmonella typhimurium* suspensions in the range of $10^4$ - $10^9$ cells/ml were used to activate the sensor. *S. typhimurium* was used in the experiments as representative *Salmonella* serovar. The concentration of bacteria was estimated by the colony count and the direct counting of cells in small samples by dark field microscopy.

Panel B. A graphical representation of the change in voltage of the *Salmonella* sensor in response to bacterial concentration. Plot of the relative change of voltage $\triangle V$ versus log *[Salmonella]* for a *Salmonella* sensor. The points correspond to experimental data ($\pm$Std. Er) and the line corresponds to the linear regression of the data. The mean value of the voltage was calculated from the V versus time graph for each solution and the $\triangle V$ was calculated as follows: $\triangle V = V_S - V_O$, where $V_S$ and $V_O$ are averages of 200 experimental data points of saturated end portions of dose-response curves for solutions with various *Salmonella* concentrations and without *Salmonella,* respectively.

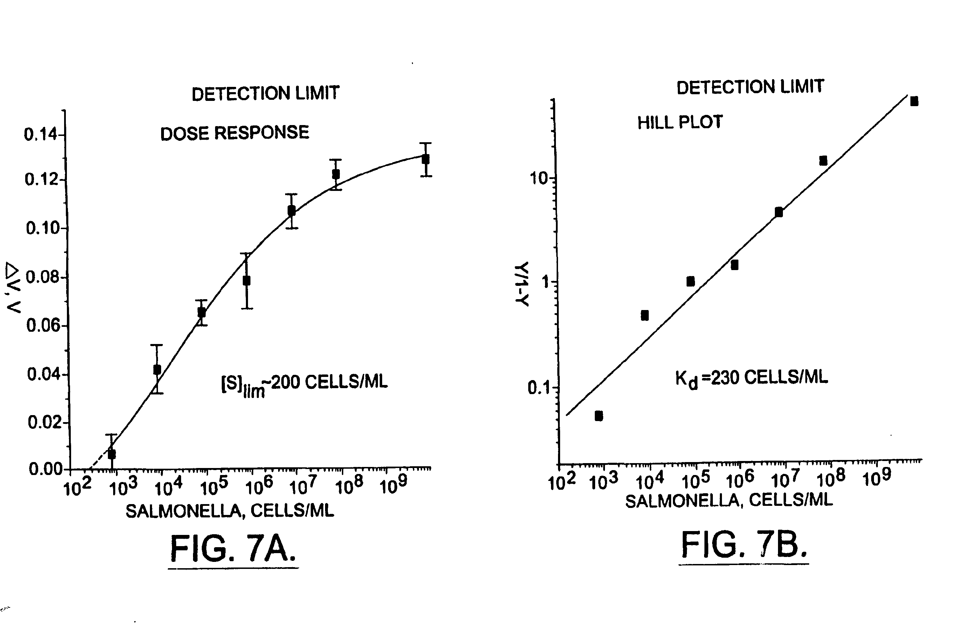

Figure 7. A graphical representation of the detection limit of the *Salmonella* sensor. Seven monolayers of the *Salmonella* O Antibody Serum were transferred to a sensor quartz crystal substrate using the Langmuir-Blodgett technique. These films were made at 19°C on a subphase containing 55mM KCl, 4mM NaCl, 1mM $MgCl_2$, 0.1mM $CaCl_2$ and 2mM MOPS with a pH of 7.4. The film covered crystal was placed into the probe cavity of the MAXTEK microbalance and 1 ml of the following solutions were applied in sequence and the change in voltage was recorded for 8 minutes for each solution at 25°C. The concentration of bacteria was estimated by the colony count and the direct counting of cells in small samples by dark field microscopy. Panel A represents the plot of the relative change of voltage $\triangle V$ versus *[Salmonella]* for a *Salmonella* sensor. The points correspond to experimental data ($\pm$Std. Er) and the curve corresponds to the Sigmoidal fit of the data. The mean value of the voltage was calculated from the sensor output (V versus time graph) for each solution and the $\triangle V$ was calculated as follow: $\Delta V = V_S - V_O$, where $V_S$ and $V_O$ are averages of 200 experimental data points of saturated end portions of dose-response curves for solutions with various *Salmonella* concentrations and without *Salmonella*, respectively. Panel B represents the Hill Plot of the corresponding data. $(\log(Y/(1-Y))=n\log[\text{bacteria}] -\log(K_d)$, where $Y=(\Delta V/\Delta V_{max})$ and $K_d$=dissociation constant of the bacteria/antibody complex).

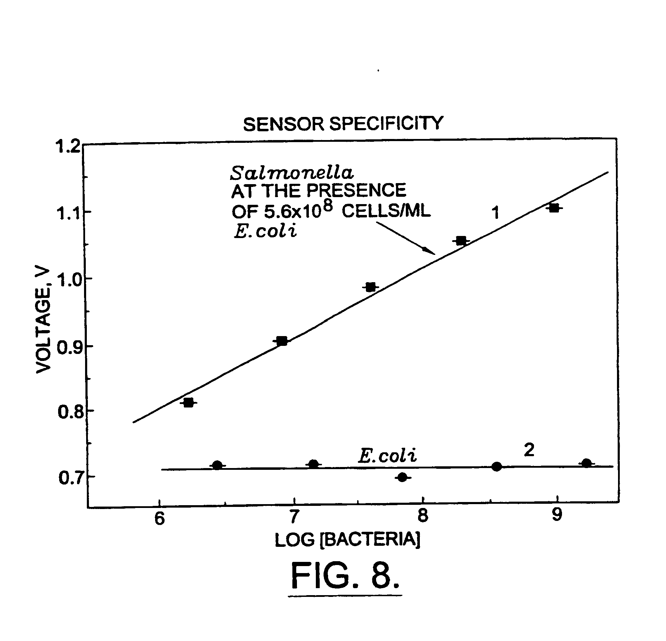

Figure 8. A graphical representation of the *Salmonella* sensor specificity.

Panel A. Line 1 represents the dose response of *Salmonella* sensor to *Salmonella* in the presence of 5.6 x $10^8$ cells/ml of *E. coli.* Line 2 shows the dose response of the *Salmonella* sensor to *E. coli* within the same range of concentration with no *Salmonella* present. For any given concentration the sensor prefers *Salmonella* over *Escherichia-coli.* A marked preference for *Salmonella* over *E. coli* is observed even when the number of *E. coli* exceeds the number of *Salmonella* by the factor of 1000.

Panel B demonstrates *Salmonella/E. coli* specificity of the sensor in the presence of undiluted unfiltered chicken liquid. The points correspond to experimental data ($\pm$Std. Er) and the line corresponds to the linear regression of the data. The mean value of the voltage was calculated from the saturated end portion of the dose-response curve of the V Versus time graph for each solution.

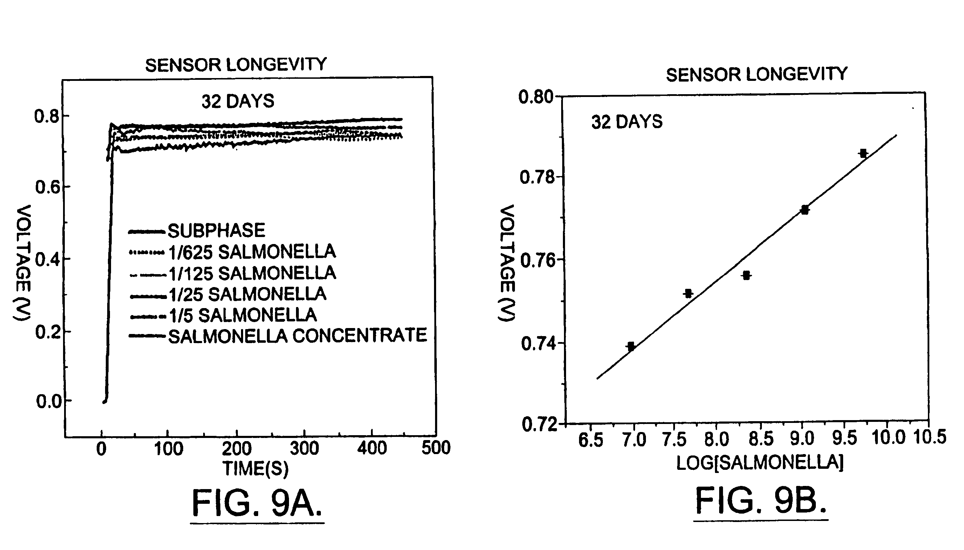

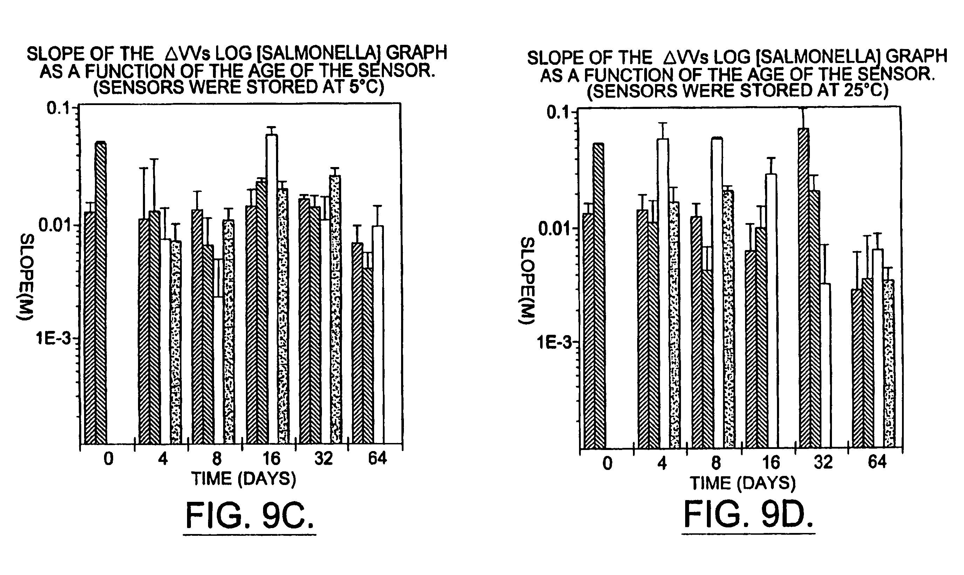

Figure 9. A graphical representation of the *Salmonella* sensor longevity.

Forty *Salmonella* antibody sensors were built by transferring 7 monolayers of *Salmonella* antibody serum to quartz crystal substrates used in the acoustic wave micro balance using the Langmuir-Blodgett technique. These films were made at 19°C on a subphase with the following composition: 55mM KCl, 4mM NaCl, 1mM $MgCl_2$, 0.1mM Ca $Cl_2$ and 2mM MOPS with a pH of 7.4 Half of the crystals were stored in a refrigerator at 5°C and the other half was stored in a dust free container at 25°C. Each set of crystals was divided into groups of 4 crystals. The 5 groups from both the refrigerated and the room temperature crystals were tested after 4, 8, 16, 32 & 64 days as described below. The film covered crystal was attached to an acoustic wave microbalance and 1 ml of the following solutions were applied in sequence and the change in voltage was measured for 8 minutes for each solution. The sensor was used to measure subphase solution, 1/625, 1/125, 1/25, 1/5 dilutions of a concentrated *Salmonella* solution and the undiluted solution. The bacteria count was measured for each time interval and the concentrations were calculated accordingly. The concentration of bacteria ranged from $10^6$ - $10^{10}$ bacteria/ml. The experiments were carried out at a temperature of 25°C. Panel A is graphical representation of the voltage with respect to time for the 32 days old *Salmonella* sensor in response to various bacterial concentrations. Panel B is a plot of mean value of voltage V versus *Salmonella* concentration for a *Salmonella* sensor calculated from the experimental data of the Panel A. The points correspond to the experimental data ($\pm$Std. Er) and the line corresponds to the linear regression of the data. The mean value of the voltage was calculated from the saturated end portion of the dose-response curve of the V versus time graph for each solution. Panel C represents the slope of the $\Delta V$ versus log *[Salmonella]* graph as a function of the age of the sensor for sensors stored at 5°C. Panel D represents the same data for sensors stored at 25°C.

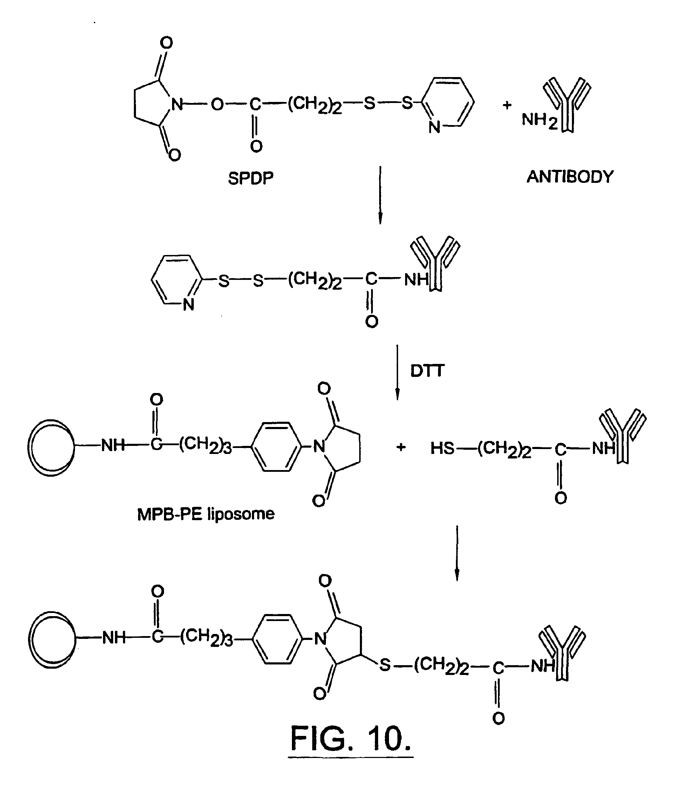

Figure 10. Schematic representation of the steps involved in the antibody-liposome conjugation.

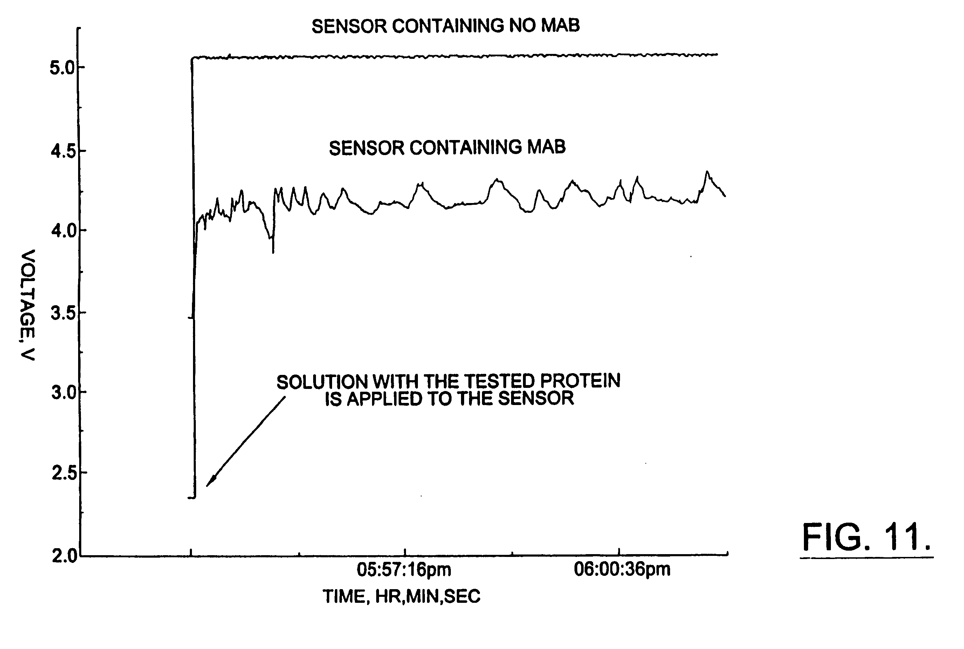

Figure 11. A graphical representation of the voltage with respect to time for sensors with and without the 5H9 monoclonal antibody, in response to a pork solution. Monolayers were prepared and transferred to sensor substrates as described in figure legend 3, for the following solutions:

(a) 80 $\mu$l of the liposome-antibody conjugate spreading solution described above; and
(b) 80 $\mu$l of the same liposome solution without antibody.

Both of the above sensors were tested with a pork solution as described in testing of bacterial sensors.

DETAILED DESCRIPTION OF THE INVENTION

Known methods for forming monolayers required dissolving an amphiphilic compound in a volatile organic solvent such as chloroform or hexane. The solvent allows the amphiphilic compound to be spread on the surface of an aqueous subphase and prevents mixing of the compound with the subphase. Upon evaporation of the organic solvent, the amphiphilic compound forms a monolayer. Inclusion of a compound of interest, such as an enzyme, antibody, or other ligand, with the amphiphilic compound results in the formation of a monolayer that may be used as a biosensor and/or chemosensor. However, the organic solvent may damage the components of the monolayer and/or leave an undesirable residue in the monolayer.

Surprisingly, an amphiphilic compound or composition may be layered onto an aqueous subphase by slowly allowing this compound or composition to run down an inclined wettable planar surface that is partially submersed into the subphase. Thus, the present invention is drawn to methods for forming monolayers without the use of organic solvents. The method comprises:

(a) providing a composition comprising at least one amphiphilic compound, wherein said composition contains not more than 25% of a volatile organic solvent and may optionally contain one or more compounds of interest;
(b) immersing one end of a wettable planar surface into an aqueous subphase, wherein said planar surface forms an angle of about 90-170 degrees to an upper surface of said subphase, wherein said subphase comprises at least one monovalent cation and at least one bivalent cation, wherein said subphase has a pH of 4.0-8.0;
(c) delivering said composition at a rate of about 0.02-4.0 ml per minute to said planar surface to form a monolayer; and
(d) compressing said monolayer.

Monolayers formed by the methods of the invention are also provided. The monolayers produced by the methods of the invention differ from prior art monolayers in that their components have not been damaged by organic solvents and have undergone self-purification and alignments during formation.

Surprisingly, the methods of the invention allow the formation of monolayers from blood serum without the addition of amphiphilic compounds or an organic solvent. Thus, the invention provides monolayers consisting essentially of serum or antiserum. The serum and antiserum may be derived from any animal. Antiserum may be raised against any immunogenic substance. Additionally, the methods of the invention allow the formation of monolayers from antibodies, bound to at least one amphiphilic compound without the addition of an organic solvent. It is recognized that an immunogenic substance may comprise an antigen, which in itself is immunogenic. Alternatively an immunogenic substance may comprise one or more haptens combined with a carrier. In preferred embodiments, an antiserum is specifically reactive with one or more antigens of microorganisms such as bacterial or viral foodborne pathogens, toxins, parasites, or meat. Bacterial antigens of particular interest are from *Escherichia coli, Salmonella, Campylobacter jejuni, Listeria monocytogenes, Staphylococcus aureus, Clostridium perfringens,* and antigens of both raw and cooked muscle foods (meat), fruits, vegetables, dairy products, juices and grains. Preferably the muscle food is pork, beef, chicken, turkey, duck or other fowl, venison, lamb, horse, seafood, fish, shrimp, or shellfish.

Langmuir-Blodgett (LB) films formed by depositing at least one monolayer of the invention onto a substrate are provided. The LB films of the invention exhibit lower background binding than prior art LB films. For example, deposition of the monolayers of the invention onto a piezoelectric crystal of an acoustic wave sensor allows detection of the binding of as little as 10 picograms (10 cells/ml of bacteria). Accordingly, a piezoelectric crystal having deposited thereon a monolayer of the invention is provided.

The monolayer in this method is formed on the air-liquid interface by allowing the spreading solution to run down a inclined wettable planar surface that is partially submersed into subphase. Figure 3 illustrates the main features of this method. The figure shows an inclined wet glass slide that is partially submersed into the subphase. Membrane vesicles (natural component of serum, or artificial lipid vesicle) are positioned on the wet slide at the edge of positive meniscus of liquid, at the liquid-air interface. The hydrophobic antibodies are bound to the vesicular membrane, while the hydrophilic antibodies and some impurities are suspended inside the vesicle. When surface and osmotic forces rupture the vesicle and it splits into monolayers, the unique process of purification occurs: membrane-bound antibodies are left bound to the newly created monolayer, but soluble antibodies and impurities go into subphase beneath the monolayer. When the monolayer is compressed and transferred onto the sensor surface, only membrane-bound antibodies remain surrounded by lipid.

Spreading material that is purified during the process of spreading needs no special preliminary purification, and sera, antisera, and a mixture of membrane-bound and soluble antibodies can be used for the stable monolayer formation. The method is simple and cost-effective.

Monolayers prepared by this method of invention can produce functional coats of high quality with properties as follows:

(a) high density of functional molecules;
(b) reactive within a large range of free ligand concentration;
(c) high specificity (the ability to the bind specific molecules in the presence of many other non-specific molecules);
(d) high sensitivity (the ratio of the change in binding to the change in the free ligand);
(e) high homogeneity (majority of binding sites have identical binding properties);
(f) high binding affinity (small dissociation constant and high detection limit);
(g) low non-specific binding;
(h) rapid attainment of equilibrium
(i) reversibility; and
(j) amplification of bacteria attachment.

Some properties of the *Salmonella* sensor produced by the invented technology are compared with those for known bacterial and viral piezoelectric immunosensors in Table 1.

| Table 1 | | | | | |
|---|---|---|---|---|---|
| Characteristics | Present invention | [1]Ye *et al.* | [2]Park and Kim | [3]Pyun *et al.* | Koing and Graetzel |
| Antigen | *Salmonella* | *Salmonella* | *Salmonella* | *E. coli* | Hepatitis virus |
| Range, cells/ml | $10^2$ - $10^{10}$ | $5.3 \times 10^5$ - $1.2 \times 10^9$ | $9.9 \times 10^5$ - $1.8 \times 10^8$ | $3 \times 10^5$ - $6.2 \times 10^7$ | $10^5$ - $10^{10}$ viruses |

Table continued

| | | | | | |
|---|---|---|---|---|---|
| Characteristics | Present invention | [1]Ye *et al.* | [2]Park and Kim | [3]Pyun *et al.* | Koing and Graetzel |
| Detection limit, cells/ml | $350 \pm 150$ | $\sim 10^5$ | $\sim 10^5$ | $\sim 10^5$ | $10^5$ |
| Time constant, min | $1.2 \pm 0.2$ | [5]25 | [5]30 - 90 | $\sim$[5]17 | 30 |
| [6]Specificity | 1:1000 | ND | ND | ND | ND |
| [6]Apparent $K_d$, cells/ml | $250 \pm 50$ | ND | ND | $3.8 \ 10^6$ | ND |
| Longevity, days | 32 | ND | ND | ND | 28 |

[1]Ye *et al.* (1997) *Journal of Food Science 62*(S):1067-1086.
[2]Park and Kim (1998) *Biosensors and Bioelectronics 13*:1091-1097.
[3]Pyun *et al.* (1998) *Biosensors and Bioelectronics 13*:839-845. [4]Koing and Graetzel (1995) *Analytica Chimica Acta 309*:19-25.
[5]Measurement time, equilibrium is not attained.
[6]Specificity over *E. coli* $K_d$-dissociation constant estimate from Hill plot; ND - not determined.

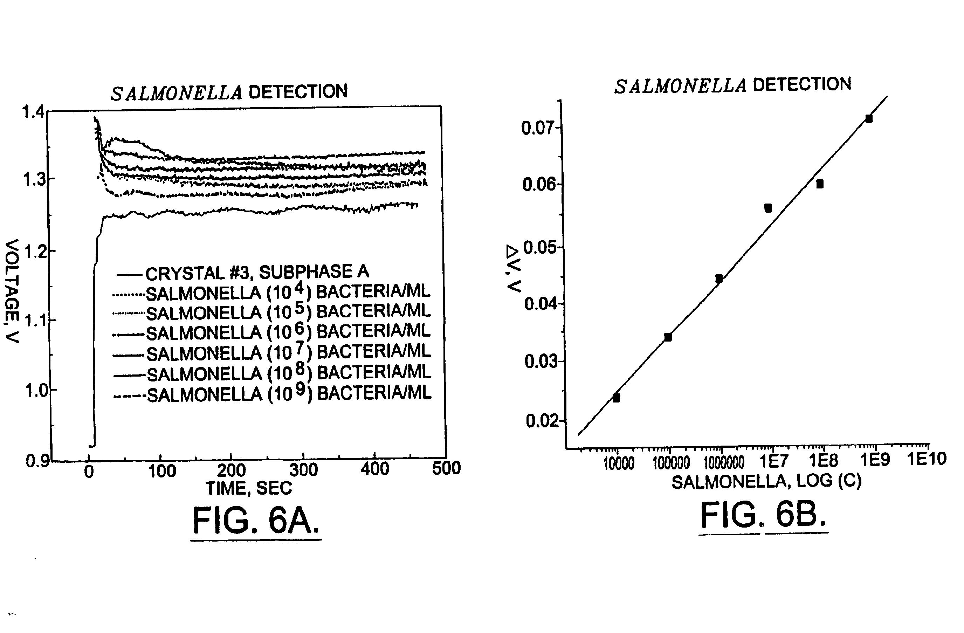

As can be seen from Table 1, performance characteristics of sensors produced by the invented method are superior to those produced by other known methods. Experimental results demonstrated in the example with the *Salmonella* sensor are consistent with the properties listed above (a-j). The high density of antibody is consistent with the large range of the *Salmonella* sensor which covers about 8 decades of bacterial concentration. High specificity and sensitivity result from the good structural alignment and environment of antibodies. The high homogeneity (majority of binding sites have identical binding properties) is consistent with the fact that a process of binding in the range of 8 decades (Figure 6) can be represented by a single line with a single dissociation constant ($K_d$). High binding affinity (small dissociation constant and high detection limit) and amplification of bacteria binding is confirmed by a very low experimental detection limit. The low non-specific binding and rapid attainment of equilibrium are also confirmed by our experimental response-time data (Figure 6, panel A).

"Amphiphilic compound", as used herein, refers to a molecule that is insoluble in water and has a hydrophilic region that will preferentially face an aqueous phase and a hydrophobic region that will preferentially face the air or a nonaqueous phase. As used herein, "amphiphilic compound" also refers to molecules that may be soluble in an aqueous solution at low concentration, but will form micelles or liposomes above a critical concentration. Many amphiphilic compounds may be used to form the monolayers of the invention. Such compounds include lipids having at least 14 carbon atoms. Examples include stearic acid and hexadecanoic acid. Other compounds that will form monolayers include, but are not limited to those described in Gaines, G.L. Jr. (1966) *Insoluble Monolayers Liquid-Gas Interface,* Interscience, New York.

Particularly preferred amphiphilic compounds are those found naturally in blood serum and those that may be used to form liposomes. Examples of compounds or compositions that will form liposomes include, but are not limited to lipids, phospholipids, such as L-$\alpha$-(1,2-dipalmitoyl-sn-glycero-3-phosphocholine) (DPPC) and maleimido phenyl butyrate phosphatidylethanolamine (MPB-PE), cholesterol, biological membranes, serum and antiserum. Preferably the amphiphilic compound is formed into a liposome prior to delivery to the wettable planar surface. Methods of forming liposomes from amphiphilic compounds and compositions are known to those skilled in the art.

"Antibody", as used herein, refers to naturally occurring antibodies, polyclonal antibodies, monoclonal antibodies, recombinant antibodies, chimeric antibodies (i.e., comprising constant and variable regions from antibodies of the same or different organism), humanized antibodies (i.e., comprising a complementarity determining region (CDR) from a non-human source), heteroconjugate antibodies (e.g. bispecific antibodies), synthetic antibodies, antigen binding forms of an antibody, such as antibody fragments such as Fab, $F(ab)_2$ and single chain Fv and derivatives thereof. An antibody specifically reactive with a particular antigen can be generated *in vivo* or by recombinant methods such as selection of libraries of recombinant antibodies in phage or other vectors. See, for example, Huse *et al.* (1989) *Science 246:*1275-1281; Ward *et al.* (1989) *Nature 341:*544-546; and Vaughn *et al.* (1996) *Nature Biotech. 14:*309-314. Methods for producing monoclonal or polyclonal antibodies are well known to those skilled in the art

The antibodies useful in the methods of the invention may be specifically reactive with any substance. Such antibodies include those specific for an antigen from a microorganism, foodborne pathogen, parasite, toxin, or meat. Particularly useful are monoclonal or polyclonal antibodies that are selective for meat proteins, preferably meat proteins from pork, poultry, chicken, turkey or duck. Antibodies specifically reactive with meat may be selective for proteins from raw meat, cooked meat or thermostable meat proteins. Particularly useful is the monoclonal antibody 5H9 that is produced by the hybridoma cell line 5H9, which was deposited on 12 December 1996 with the American Type Tissue Collection, Rockville, MD, USA (ATCC Accession No.:HB-12245). Also useful are polyclonal or monoclonal antibodies having the specificity of monoclonal antibody 5H9.

"Antigen", as used herein, refers to a substance to which an antibody can be generated and/or to which the antibody is specifically immunoreactive. It is recognized that an antigen may react with an antibody, yet not be capable of eliciting an immune response.

"Antiserum", as used herein, refers to serum that contains antibodies specific for one or more antigens.

"Aqueous", as used herein, refers to a solution in which water is the solvent

"Cation", as used herein, refers to any positively charged atom. Examples of bivalent cations useful in the subphase solution include, but are not limited to calcium, cadmium and magnesium. Examples of monovalent cations useful in the subphase solution include, but are not limited to sodium and potassium.

"Compound of interest", as used herein, refers to any molecule soluble in a compound or composition that can be formed into a monolayer. A compound of interest will ordinarily be added to the compound or composition to be formed into a monolayer prior to spreading of the monolayer on the subphase surface. Alternatively, a compound of interest may be covalently or noncovalently attached to a liposome prior to formation of the monolayer. In addition, compounds of interest could be subsequently attached, covalently or noncovalently to the monolayer. Compounds of particular interest include, but are not limited to polypeptides such as enzymes, monoclonal and polyclonal antibodies, receptors and other peptide ligands, nucleic acids, lipids and carbohydrates. Preferably, the compound of interest specifically binds a microorganism, virus, parasite, toxin, foodborne pathogen, meat or a component thereof.

"Compressing", as used herein, refers to moving one or more compression barriers of a Langmuir-Blodgett apparatus so as to reduce the surface area in which the monolayer has formed. As this surface area decreases, the intermolecular distance decreases and the surface pressure increases. This relationship may be graphically represented by an isotherm, which plots the surface pressure versus the area per molecule.

"Delivering", as used herein, refers to any method used to apply the composition to be formed into a monolayer onto the planar surface. Preferably, the composition is delivered to the planar surface using a micropipette. However, those skilled in the art will know of variety of delivery options that may be used in the methods of the invention. The rate of delivery of the composition to the planar surface will be about 0.02-4.0 ml per minute, preferably about 0.05 - 0.75 ml per minute, and most preferably about 0.1 ml per minute.

"LB Film", as used herein, refers to a monolayer that resides on the surface of a substrate. An LB Film may be formed on any surface. A preferred surface is a piezoelectric crystal.

"Muscle Food", as used herein refers to both raw and cooked meats from any animal. Muscle Foods of particular interest include those of game animals, fish, seafood, and animals raised for human consumption. Preferably, the muscle food is pork, beef, chicken, turkey, duck or other fowl, venison, lamb, horse, fish, seafood, or shellfish.

"Microorganism", as used herein refers to any organism that is not visible by the human eye without magnification. Microorganisms may exist as single cells or cell clusters, or chains, and include, but are not limited to viruses, bacteria, cyanobacteria, protozoa, algae and fungi. Microorganisms of particular interest include *Escherichia coli, Salmonella, Campylobacter jejuni, Listeria monocytogenes, Staphylococcus aureus* and *Clostridium perfringens.*

"Monolayer'', as used herein, refers to a one molecule thick film of at least one amphiphilic compound or composition. Optionally, one or more compounds of interest may be included in the monolayers of the invention.

"Piezoelectric", as used herein, refers to the ability to generate a voltage when mechanical force is applied, or to generate a mechanical force when voltage is supplied. This reciprocal relationship is referred to as the piezoelectric effect. The absence of a center of symmetry in the piezoelectric crystal is necessary for the piezoelectric effect. Of the 21 classes of crystals that lack a center of symmetry, all but one class are piezoelectric. A preferred piezoelectric crystal is a quartz crystal.

Application of a voltage across a piezoelectric crystal having an LB film deposited thereon, produces a mechanical vibration of a certain resonance frequency. A change in the mass of the crystal resulting from an interaction of substances in solution with a component of the LB film changes the resonance frequency. This change in resonance frequency may be measured as a change in voltage. The change in voltage is proportional to the concentration of the specifically binding substance, provided that nonspecific binding is low.

"Serum", as used herein, refers to the fluid portion of blood obtained after removal of the fibrin clot and blood cells. Serum may be prepared from the blood of any animal, including humans.

"Specifically reactive", as used herein, refers to a binding reaction where two or more interacting substances have at least a two fold greater affinity for each other than for background components.

"Substrate", as used herein, refers to any non-soluble solid on which an LB film may be formed. Such solids include: quartz, glass, mica, plastic, some metals (chrome, gold, silver). A preferred substrate is a piezoelectric crystal, most preferably a quartz crystal.

"Subphase", as used herein, refers to an aqueous solution onto which the composition to be formed into a monolayer is spread. At least one bivalent and one monovalent cation must be present in the subphase. Suitable subphase include, but are not limited to those described by Gains, ("Insoluble monolayers at liquid-gas interface." (1996) Interscience, New York). A typical subphase comprises: 55.0 mM KCl, 4.0 mM NaCl, 1.0 mM $MgCl_2$, 0.1 mM $CaCl_2$ and 2.0 mM MOPS buffer in deionized doubly distilled water, pH 7.4. The subphase is placed in the trough of the Langmuir-Blodgett apparatus prior to spreading the monolayer.

"Volatile organic solvent", as used herein, refers to an organic liquid that is nonmiscible with water, has a density less than 1.0, a boiling point of less than 100°C and is capable of dissolving an amphiphilic compound. Examples of volatile organic solvents include chloroform, hexane, benzene, decane and ether.

"Wettable", as used herein, refers to a surface to which a liquid will adhere. Examples of wettable surfaces include, but are not limited to glass, silicon and mica. A preferred wettable planar surface is a disposable glass microscope slide.

Typically, the monolayer will be formed in a Langmuir-Blodgett apparatus. Such apparatuses are well known in the art and are commercially available. For example, in the work described below, a KSV 2200LB Langmuir-Blodgett apparatus (KSV-Chemical, Finland) was used. The LB apparatus usually comprises at least one trough, a moving compression barrier that allows regulation of the surface pressure of the monolayer, and a device that measures the surface pressure. The trough holds the subphase solution and is typically made of Teflon™. Motors may move the compression barrier and raise and lower the substrate through the monolayer. Most troughs are fully automated and also are temperature regulated and vibration-controlled.

Insoluble monolayers prepared at the air/water interface are extremely sensitive to various factors such as temperature, pH, certain metal ions, surface active contaminants and other contaminants that collect at the air/water interface. The total amount of material in a typical monolayer is about one microgram. Consequently, impurities in the order of parts per billion can cause serious problems if they collect at the air/water interface. Thus, careful attention to experimental detail and procedures is required for all monolayer and LB film work.

Methods of forming monolayers are known to those skilled in the art. See, for example, Roberts, *supra*. The critical difference between the methods of the invention and the prior art methods is the manner in which the compound or composition to be formed into a monolayer is delivered to the surface of the subphase. The objective of both the prior art and instant delivery method is to place the composition to be formed into a monolayer at the air/surface interface of the subphase without dissolving this composition, or components thereof, in the aqueous subphase. The prior art method accomplishes this objective by dissolving the composition in an organic solvent, which forms a separate phase at the air/water interface, and then evaporates to leave a monolayer of the amphiphilic composition. In contrast, the delivery method of the instant invention does not require the use of an organic solvent. Surprisingly, an amphiphilic compound or composition may be layered onto an aqueous subphase by slowly allowing this compound or composition to run down an inclined wettable planar surface that is partially submersed into the subphase.

One end of the wettable planar surface is placed into the subphase at an angle of about 90-170 degrees to the subphase surface (Figure 1). The angle will affect the rate and force at which the composition to be formed into a monolayer is delivered to the surface of this subphase. The force must be sufficiently low so as to avoid mixing of the composition with the subphase. Preferably, the angle will be about 140-170 degrees, most preferably, about 165 degrees. Other factors affecting the rate and force of delivery of the composition to the subphase surface include the makeup of the composition to be formed into a monolayer, the rate at which this composition is delivered to the planar surface, the planar surface and the subphase. It is critical that the composition to be formed into a monolayer contains lipid forming vesicles. The osmolarity of the vesicular liquid must be greater than that of the subphase.

One skilled in the art of monolayer formation will be able to empirically determine the angle and delivery rate best suited to a particular application. The following criteria should be applied to assess the efficiency of the spreading procedure:

1. The loss of the spreading material should be minimized. The loss can be estimated by the recovery coefficient defined as $R = M_m / M_s$, where $M_s$ is the mass of the substance in the spreading solution, and $M_m$ is the mass of the monolayer. In the successful spreading procedure the R should be close to 1.0. An R<1 indicates losses of the substance. For example, R=0.5 would indicate 50% loss of the spreading material.

2. The spreading procedure should not cause significant changes in physical chemical activity of the spread substance. For example, at the very low rate (<0.01 ml/min) and in the absence of lipids, proteins may produce monolayers with unfolded and denatured molecules.

Compression of a monolayer results in a transition from a gas phase to a liquid phase. Additional compression results in a transition from a liquid phase to a solid phase in which the molecules of the monolayer form a tightly packed, ordered structure. Further compression results in a collapse of the monolayer due to mechanical instability and a concomitant decrease in surface pressure. If the monolayer has more than one component, for example an antibody component, there may be a first collapse pressure at which the antibodies collapse and a second higher collapse pressure at which the rest of the monolayer collapses. Graphing of the surface pressure in response to movement of the compression barrier produces an isotherm that may be used to determine the optimal compression for a particular monolayer under a particular set of conditions. The optimal surface pressure is achieved just before a pressure is reached that results in the collapse of one or more monolayer components.

After the desired surface pressure is achieved by compression of the monolayer, an LB film may be formed by passing a substrate through the monolayer one or more times. Methods of forming LB films are known to those skilled in the art and are described in Ollman, *supra* and Roberts *supra*.

The following examples are intended to illustrate, rather than to limit the invention.

EXPERIMENTAL

**Materials and Methods**

**Cultures.** *Salmonella typhimurium* and *Escherichia coli* 0157:H7 are used in the experiments. Both cultures are obtained from the Auburn University Botany & Microbiology Department's culture collection. Each one is confirmed for identity through the use of traditional biochemical, cell morphology, and serologic tests. The cultures are maintained on Trypticase agar (TSA) slants.

**Growth of cultures for experiments.** Each culture is streaked for isolation on TSA plates before inoculation of a fresh culture to trypticase soy broth for overnight incubation at 37°C in a shaking water bath incubator. Then, the cells are washed by performing centrifugation (3500 rpm for 10 minutes) of the broth and resuspending the cells in 10 mL sterile phosphate buffered saline (PBS) (pH 7.0) before repeating the centrifugation step and resuspending in 2 mL PBS. Aseptic procedures are used throughout the steps taken to produce the test volumes of bacteria.

**Dilutions.** Serial dilutions are made with PBS by initially adding 8 mL diluent to the cells suspended in 2 mL (1:5) of the liquid. Then 3 further dilutions are made by serially transferring 2 mL to 8 mL diluent for 1:25, 1:125, and 1:625 dilutions. Then, further dilutions are made by serially transferring 1 mL to 9 mL diluent in 4 steps to accomplish a 1:10 dilution of each tube. All tubes are shaken before each pipetting to assure mixing before delivery.

**Colony forming unit (cfu) determinations.** The number of viable cells in each dilution is determined by spread plating 0.1 mL of each dilution onto duplicate plates of TSA, and incubating 48 hours before making a final count of the cfu's and calculating the average cfu based on dilutions yielding 30-300 colonies. The tubes with diluted cells are immediately placed on ice and delivered to another laboratory for testing on the sensor.

**Antibodies.** Antibodies used as capture antibodies on the membranes attached to the sensor are obtained from Oxoid, Inc. (Ogdensburg, NY). For *S. typhimurium,* a polyvalent somatic O antibody specific for most *Salmonella* serovars is employed. To capture *E. coli* O157:H7, either polyvalent 0157 (outer membrane) or H7 (flagellar) antibodies are used. In most cases, the same lot of antibody is used. Reactivity is checked against the target bacterium by performing a slide agglutination test in which the cells agglutinated in the presence of the specific antibody. Monoclonal Anti *E. coli* 0157 and Monoclonal *Salmonella typhimurium* are obtained from Biodesign International (Kennebunk, ML).

**Procedures for examining the reactivity of antibodies**. (1) Dot blot ELISA test using nitrocellulose or nylon filters with antigen fixed and subjected to chromogenic anti-mouse (for monoclonal antibody) or anti-animal-based antibody conjugated with enzyme assay. (2) Kit tests for target organisms. (3) Agglutination tests.

**Construction of lipid vesicles containing antibodies and Fabs.** Large, unilamellar liposomes are prepared from synthetic L-$\alpha$-(1,2-dipalmitoyl-sn-glycero-3-phosphocholine) (DPPC) and maleimido phenyl butyrate phosphatidylethanolamine (MPB-PE). The monoclonal antibodies (or Fabs) with specificity for *S. typhimurium* or *E. coli* O157:H7 are modified by the heterobifunctional reagent N-Succinimydyl-3(2-pyridyldithio)propionate (SPDP) in the presence of Dicetyl Phosphate and Dithiothreitol (DTT) and are conjugated to liposomes (Betageri *et al.* (1993) *J. Pharm. Phamacol. 45*:48-53). Liposomes containing specific antibodies are converted into monolayers and deposited on the sensor surface by LB method.

**Monolayer techniques.** Surface Film Balance. Measurements of surface pressure are performed on a Langmuir-Blodgett film balance KSV 2200 LB (KVS-Chemicals, Finland). The fully computerized system contains a Wilhelmy-type surface balance (range 0-100 mN/m; sensitivity 0.05 mN/m), a Teflon trough ($45 \times 15$ cm$^2$), a variable speed motor-driven Teflon barrier (0-200 mm/min), and a laminar flow hood. The trough is mounted on a 200 kg marble table. Vibration control is provided by interposing rubber shock absorbers, and by mounting the laminar flow hood on a separate bench. Surface pressure is monitored by use of a sandblasted platinum plate of 4 cm perimeter.

Temperature of the subphase is controlled ($\pm 0.1$°C) by water circulation through a quartz tube coil on the bottom of the trough. Temperature is measured by a thermistor located just below the water interface. Surface pressure data are collected during slow, steady-state compression of the monolayers.

**Free energy, enthalpy and entropy.** The thermodynamic value of free energy, enthalpy, and entropy derived from the isothermal compression data are calculated by using the following equations (Ito *et al.* (1989) *Thin Solid Films 180:1-13;* Vodyanoy *et al.* (1990) *Biochim Biophys Acta 1047*:284-289; Vodyanoy *et al.* (1994) *Langmuir 10*:1354-1357; Pathirana *et al.* (1992) *J. Am. Chem. Soc. 114*:1404-1405, Pathirana *et al.* (1992a) *Langmuir 8*:1984-198, Pathirana *et al.* (1996) *Supramolecular Science 3*:149-154, Pathirana *et al.* (1998) *Langmuir 14*:679-682.

$$(\Delta G) = \int_{J^{P-0}}^{P=x} A dP$$

$$\Delta H = \Delta G + T\Delta S$$

$$(\Delta S)_P = -[\partial(\Delta G)/\partial T] + (\partial c_w/\partial T)_P(A_{P=0}-A_{P=x})$$

$$c_w = 75.680 - 0.138t - 3.56*10^{-4}t^2 + 4.7*10^{-7}t^3,$$

where $\Delta G$, $\Delta H$ and $\Delta S$ are free energy, enthalpy and entropy of compression; $c_w$ is the surface tension of pure water, A and P are surface area and pressure, and T is the absolute temperature and t is the temperature ($\Delta C$).

**Surface potential.** Surface potentials are measured with a $^{210}$Po air electrode located 2 mm from the water surface connected to an electrometer, and referenced to an Ag-AgCl electrode immersed in the subphase. Surface potential V and area A isotherms are measured simultaneously with the surface pressure isotherms and are used for calculations of the surface dipole moments $\mu$ from the equation $\mu=AV/12\pi$, where A($Å^2$/molecule) is the molecular area, V is in millivolts, and $\mu$ is in millidebye units (Gaines, G.L. Jr. (1996) *Insoluble Monolayers at Liquid-Gas Interface,* Interscience, New York and Pathirana *et al.* (1992) *J. Am. Chem. Soc. 114:1404-1405).*

**Elasticity.** The monolayer elasticity $E=-A(\partial P/\partial A)_r$ as a function of the surface pressure, is calculated directly from the pressure isotherms (Vodyanoy *et al.* (1990) *Biochim Biophys Acta 1047*:284-289).

**Viscosity.** The surface viscosity of the monolayers is measured by the canal viscometer by replacing the solid compression barrier of the LB trough with the one containing a 0.265 x 2.0 $cm^2$ slit. The monolayer is allowed to flow through a slit in the water surface, from a region of surface pressure, $P_2$, to one where the surface pressure has a lower value, $P_1$. Jody's formula (Gaines, 1966) is used for calculation of the surface viscosity, ($\eta_s$):

$$Q = (P_2 - P_1)/(cl\eta_0)[a-2(\eta_s/c\eta_0)^{1/2}\tanh(c\eta_0/\eta_s)^{1/2}a/2]$$

where a and 1 are the width and length of the canal, Q is the area of monolayer flowing through the canal per second, $\eta_0$ is the bulk viscosity of the subphase liquid, and $c\eta_0 = 0.191$ is a device constant.

**Monolayer formation.**

*Phospholipid monolayers.* Phospholipid solution is spread on the surface balance as hexane solutions (1 mg/ml) containing 2% ethanol (Ito *et al.* (1989) *Thin Solid Films 180:1-13).* After compression the monolayers are recovered for phosphate analysis (Bartlett, G.R. (1959) *J. Biol. Chem. 234*:466-468).

*Monolayers made of antibody preparations.* Monolayers containing antibodies are prepared and spread by the following methods of the instant invention.

**Monolayer deposition.** Monolayers containing antibodies are transferred at a constant surface pressure onto a round (d=2.5 cm) quartz plate with gold electrode for microbalance measurements; or 50×10×3 mm, 45° face angle, germanium parallelogram attenuated total reflection (ATR) plates (Wilmad) for FTIR spectra measurements; or standard microscope slides for visual observations and image analysis (Pathirana *et al.* (1992) *J. Am. Chem. Soc. 114*:1404-1405).

**Bacteria binding measurements.** QCM crystals. AT-cut planar quartz crystals with a 5 MHz nominal oscillating frequency are supplied by Maxtek, Inc. Circular gold electrodes are deposited on both sides of the crystal for the electrical connection to the oscillatory circuit.

Quartz crystal microbalance. Mass measurements were carried out with a PM-700 Maxtek microbalance with a frequency resolution of 0.5 Hz at 5 MHz, and a mass resolution of 10 ng/cm$^2$. The device is capable of working in both single and dual probe modes. A microbalance is interfaced with the computer and used as a monitoring device to record binding of bacteria to the sensor surface. The sensor substrate covered with the antibody film is positioned in the probe arm of the instrument just before delivery of test solutions. Immediately after the recording is started, approximately 1 ml of the control solution is delivered with a pipette to the sensor surface and the voltage is recorded for 4 - 8 minutes. The test solution is then carefully removed with a plastic pipette tip. After removal of the control solution, a new recording is initiated and 1 ml of the same solution containing bacteria is added and the same measuring procedures followed. After all the solutions are tested, the sensor crystal is carefully removed and is placed in absolute ethanol until it is cleaned in nitric acid. All the equipment used in the experiment are sterilized in absolute ethanol each day. The data collected are stored on a hard disk and analyzed off line using Lotus 123 and Microcal Origin software.

Calibration: The Quartz crystal microbalance is calibrated by the deposition of well characterized stearic acid monolayers. The deposition of a single monolayer of the stearic acid onto a QCM crystal adds an additional mass of 2.6 x 10$^7$ g/cm$^2$ (for a 30 mNm$^{-1}$ transfer surface pressure).

**Dark-field microscopy**. Optical observation of bacteria attachment is viewed with an Olympus microscope fitted with a 100-W mercury lamp illumination source, a polarizer, a Naessens dark-field condenser (COSE Corp., Canada) and a 100x objective lens (oil, NA 1.4) (Pathirana *et al.* (1996) *Supramolecular Science 3*:149-154). The dark-field images (Inoue *et al.* (1997) *Video Microscopy,* Plenum Press, New York) are directed to a DEI-470T Optronics CCD Video Camera System (Optronics Engineering, CA), captured with a Pentium DEL computer using a 1.0 Pro Series Capture Kit and a 2.0 Image-Pro Plus software (Media Cybernetics, MD). The images are displayed on a video monitor (PMV-1343 MD, Sony Corp.) and hard copied with a video recorder (SVO-9500MD, Sony Corp.) and printer (UP 3000, Sony Corp.). This system provides real time direct-view optical images of high resolution. No freezing, dehydration, staining, shadowing, marking, or any other manipulation of the samples was required; they can be observed in the natural aqueous environment (Vodyanoy *et al.* (1992) *J. Am. Chem. Soc. 114*:1404-1405). A direct count of bacteria is used to determine their concentration in liquid samples. The count of bound bacteria in conditions of large general concentrations of motile cells is used to estimate the surface concentration of a specific antibody.

**Binding equations**. The purpose of this section is to construct a quantitative description of binding by using known binding equations and to describe the interaction of antigens and antibodies and resulted complexes in terms of binding parameters. The applicability of the binding equation to the antibody-antigen system is not trivial and requires special considerations. The reaction between antigen molecules and an antibody can be schematically presented as

$$nAg + Ab \leftrightarrow AbAg_n \tag{1}$$

where n is the number of molecules of antigen bound to a single antibody. The association binding constant ($K_b$) for this reaction can be defined as (Kuchel *et al.* (1988) *Theory and Problems of Biochemistry,* McGraw-Hill, New York).

$$K_b = [AbAg_a]/[Ab][Ag]^n \tag{2}$$

If we neglect the number of antibodies bound to nonspecific molecules then the total number of antibodies ($C_{Ab}$) is composed of free antibodies and antibodies bound to antigen molecules:

$$C_{Ab} = [Ab] + [AbAg_n] \tag{3}$$

Combining Eqs (2) and (3) we can determine the fraction of antibodies occupied by antigens:

$$Y = [AbAg_n]/C_{Ab} = K_b[Ag]^n/(1 + K_b[Ag]^n) \qquad (4)$$

The ratio of occupied and free antibodies can be defined as

$$Y/(1-Y)=K_b[Ag]^n \qquad (5)$$

Taking the logarithm of both sides, we get

$$\log(Y/(1-Y))= \log K_b + n\log [Ag] \qquad (6)$$

A plot of the left-hand side of Eq. (6) versus log [Ag] is known as a Hill plot. It gives an estimate of n from the slope, $K_b$ from the ordinate intercept, and $EC_{50}$ at the point when Y=1-Y (or Y=0.5). If antibodies are immobilized in the membrane interfaced with an antigen, then the concentration of the antibody/antigen complexes is directly proportional to the change of the membrane mass due to the transfer of antigen molecules from solution to membrane, which we denote $\Delta m$ and the maximal mass change is proportional to the total number of bound antigen molecules:

$$[AbAg_n] \sim \Delta m \sim \Delta V \qquad (7)$$

$$C_{Ab} \sim \Delta m_{max} \sim \Delta V_{max} \qquad (8)$$

where $\Delta V$ is the output voltage change of the QCM device reflecting the change of mass. Thus, in experiments with measurements of antibody binding as a function of antigen concentration, we can analyze results using Equations (4-6) by substituting $Y = [AbAg_n]C_{Ab}$ with

$$Y = \Delta m/\Delta m_{max} =\Delta V/\Delta V max \qquad (9)$$

If antibody binds a single molecule of antigen (n=1), then from Eqs (4) and (9) we can estimate the binding activity for a single molecule of antigen:

$$\Delta V/[Ag]= - (1/K_d) \times \Delta V + (1/K_d) \times \Delta V_{max} \qquad (10)$$

where $K_d = 1/K_b$ is the apparent dissociation binding constant of the antigen-antibody complex. Equation 10 can be presented as linear plot of the left-hand side versus the voltage response, $\Delta V$. The plot is called a Scatchard Plot *(Scatchard et al.* (1949) *J. Am. Chem. Soc. 79*:12-20). It yields an estimate of $-1/K_d$ from the slope and $\Delta V_{max}$ from the abscissa intercept. Non-specific binding can be estimated by two ways: 1) From the saturation curve for an antigen binding to a homogeneous antibody population. The total binding includes a component of non-specific binding (NSB), which is non-saturable, and the reminder is specific binding, which saturates at $\Delta V_{max}$; and (2) From competitive binding assay (Kuchel *et al.* (1988) *Theory and Problems of Biochemistry,* McGraw-Hill, New York). The NBS is estimated as a fraction, which is not displaced by other ligands for the antibody.

**Example 1**

Preparation of Monolayers and LB Films from Anti-Bacterial Immunosera

Preparation of the Langmuir-Blodgett Apparatus

Monolayers were prepared using a KSV 2200LB Langmuir-Blodgett apparatus (KSV-Chemical, Finland). The LB apparatus was thoroughly cleaned according to the following procedure. Prior to the monolayer preparation, the Teflon trough and the compression barrier of the LB apparatus were cleaned with either absolute ethanol or hexane. Special care was taken to ensure that the edges of the trough, where contaminants can collect, were thoroughly cleaned. The trough was then filled with distilled water until a positive meniscus of about 2 mm formed at the trough edges. Contaminants floating at the air/water interface were removed by sweeping the compression barrier over the surface of the trough 3 times. Water that overflowed into the gutter was removed via a thin vinyl tube connected to a suction pump. The water in the trough was completely removed by suction immediately before filling the trough with the subphase. The enclosure doors were closed whenever possible to reduce contamination.

After cleaning the trough as described above, the trough was filled with 1.5 L of subphase solution comprising: 55.0 mM KCl, 4.0 mM NaCl, 1.0 mM $MgCl_2$, 0.1 mM $CaCl_2$ and 2.0 mM MOPS buffer in deionized doubly distilled water. The pH of the resultant solution was adjusted to 7.4 with 1N KOH. The solution was then filtered, stored in a glass bottle and used within 2 days.

The trough thermostat was then adjusted to 20°C. When the subphase reached this temperature ($\pm$0.1 °C), the subphase surface was cleaned 3 times by sweeping with the compression barrier. The overflow was removed from the gutter by suction.

The Wilhelmy plate was cleaned by successive sonication in the following solvents for I minute each:

1. 2:1 chloroform/methanol
2. acetone
3. doubly distilled deionized water
4. absolute ethanol

In order to ensure that the Wilhelmy plate was completely wettable, it was sonicated in doubly distilled deionized water immediately before placement in the hang wire of the LB apparatus.

The pre-cleaned Wilhelmy plate was then suspended perpendicular to the subphase surface so that 1/3 of the plate is dipped in the subphase. In this arrangement, the $^{210}$Po air electrode was kept 3 mm from the subphase surface and the Pt plate was completely wetted. The compression barrier was fitted into the traverse fork and the surface pressure and the surface position were zeroed using the DFC unit.

Formation of the Monolayers

A glass microscope slide (1 × 2 inch) was fixed to a sample holder that held the slide at a 165° angle to the subphase surface (Figure 6). The slide was then positioned so that its lower edge was just under the surface of the subphase.

Commercially available antisera to *Salmonella* or *Escherichia coli* were used as purchased for the preparation of monolayers. *E. coli* H (H-7) and *Salmonella* O (polyvalent O) antisera from Denka Seiken CO., LTD, Tokyo, Japan, were used as purchased for monolayer preparation. *Salmonella* antiserum (In Vitro Diagnostic Reagent No. 1438) is of rabbit origin, possessed O-grouping, H and Vi antigens, and was prepared in liquid form for serological studies of *Salmonella* and for the determination of sero types of *Salmonella* by slide agglutination and test tube agglutination. The antiserum was mixed well and then, using a micropipette, 100 μl was deposited drop-wise onto the surface of the microscope slide at a rate of about 0.1 ml/minute. The microscope slide was next immersed in the subphase so that the area of the slide covered by the antiserum was completely submersed. The slide was then withdrawn completely from the subphase solution. After 10 minutes, the monolayer was compressed to a surface pressure of 23 mN/m. The pressure is determined by analysis of surface pressure-surface area isotherms.

LB Film Deposition

Unpolished, 5 MHz, AT-cut quartz crystals with a piano-piano configuration were obtained from Maxtek, Inc. and were used as substrates in these experiments. The crystals are 1 inch in diameter with a gold electrode over a special adhesion layer. The sensor substrates were cleaned according to the following procedure. The substrates were placed in 50% nitric acid for 2 days and then rinsed thoroughly in distilled water to remove any traces of nitric acid. The substrates were then dried in air and stored in covered containers until further use. Before spreading the monolayer as described above, the substrate was fixed to a sample holder and placed in the subphase so that the area to be covered by the monolayer was under the subphase surface.

The antisera monolayers were deposited as X type films with a transfer ratio of 1 $\pm$ 0.1. The substrates are hydrophilic and therefore, deposition of the first monolayer occurs when the substrate is withdrawn from the subphase through the compressed monolayer. The surface pressure of the monolayer was kept at a constant value throughout the film deposition process.

## Example 2

Quantitation of *Salmonella* Using a Piezoelectric Crystal Having an LB Film Comprising *Anti-Salmonella* Serum

The sensor substrate and attached monolayers of *anti-Salmonella* serum described in Example 1 were used with a sensor electronics package to measure the change in resonance frequency in response to dilutions of *Salmonella* ranging from $10^2$ to $10^9$ bacteria/ml.

A Maxtek, Inc., PM-700, plating and etching monitor was interfaced with the computer and used as a monitoring device to record binding of bacteria to the sensor surface. The sensor substrate covered with the antibody film was positioned in the probe arm of the PM-700 instrument just before delivery of test solutions. The sensitivity of the device was set to 1V/$\mu$m and output voltage was set to zero. Immediately after the recording started, 1 ml of the control solution [55mM KCl, 4mM NaCl, 1mM $MgCl_2$, 0.1 mM $CaCl_2$, and 2mM MOPS with a pH of 7.4] was delivered with the 1000 $\mu$l Wheaton pipette to the sensor surface and the voltage was recorded for 8 minutes. Then the test solution was carefully removed with a plastic pipette tip. After removal of the control solution a new recording was initiated and 1 ml of the same solution containing the lowest concentration of bacteria was added and the same measuring procedures were followed. These procedures were repeated for all bacteria solutions. After all the solutions were tested, the sensor crystal was carefully removed and was placed in absolute ethanol until it was cleaned in nitric acid. All the equipment used in the experiment was sterilized in ethanol each day. The data collected were stored on hard disk and analyzed off line using Lotus 123 and Microcal Origin softwares.

The change in voltage of this *Salmonella* sensor was proportional to the log concentration of *Salmonella* (Figure 6B). The lowest detection limit of this measuring system was estimated from dose-response curves (Figure 7A) and Hill plots (Figure 7B) (see Hill, A.V. (1913) *J. Biochem.* 7:471) and was about 200 cells/ml. The signal from the *Salmonella* sensor reached equilibrium within 100 seconds after exposure to a wide range of concentrations of *Salmonella* in solution (Figure 6A). In addition, the *Salmonella* sensor was specific for *Salmonella,* giving no signal when exposed to up to $10^9$ *E. coli*/ml (Figure 8). Line 1 represents the dose response of the *Salmonella* sensor to *Salmonella* at the presence of $5.6 \times 10^8$ *E. coli*. Line 2 show the dose response of the *Salmonella* sensor to *E. coli* within the same range of concentration with no *Salmonella* present. For any given concentration, the sensor is specific for *Salmonella*. A marked specificity for *Salmonella* over *E. coli* is observed even when the number of *E. coli* exceeds the number of *Salmonella* by the factor of 1000.

## Example 3

Stability of Serum Monolayer Films

A series of *Salmonella* sensors were prepared for longevity tests. A few sensors were tested immediately after preparation. The rest of the series was divided into two groups. One group was refrigerated at 5°C and another one was stored in a dust free container at 25°C. Each set of crystals were divided into groups of 4 crystals each. The 5 groups from both the refrigerated and the room temperature crystals were tested for *Salmonella* after 4, 8, 16, 32 & 64 days, respectively. After 32 days, the sensor retained its ability to accurately quantitate bacteria in less than 30 seconds (Figure 9A). In addition, the signal remained proportional to the log concentration of bacteria (Figure 9B). The results of these experiments for all sensors are summarized in Figures 9C and D, which represent the slopes of the $\triangle$V versus log *[Salmonella]* graphs for the two groups stored at 5 and 25°C. The data show that sensors retained their sensitivity for 32 days, but had somewhat decreased sensitivity after 64 days of storage.

## Example 4

Preparation of Antibody-Coupled Liposome Monolayers and Deposition onto Sensor Substrates

Antibodies were coupled to liposomes according to the following procedure developed by Betageri *et al.* (1993) *J. Pharm. Pharmacol. 45*:48-53.

## <u>Materials</u>

Synthetic L-$\alpha$-1,2-dipalnutoyl-sn-glycerol-3-phosphocholine (DPPC) and maleimido phenyl butyrate phosphatidylethanolamine (MPB-PE) were obtained from Avanti Polar Lipids, Inc. (Alabaster, AL). Cholesterol, dicetyl phosphate and dithiothreitol (DTT) were purchased from Sigma Chemical Co. (St. Louis, MO). N-Succinimydyl-3-(2-pyridyldithio) propionate (SPDP) was purchased from Pierce, Rockford, IL. The 5H9 monoclonal antibody specific to thermostable pork skeletal muscle protein (24 KD) was prepared and purified in the laboratory of Dr. Peggy Hsieh (Dept. of Nutrition and Food Science, Auburn University, Alabama).

Preparation of Liposomes

One ml of large, unilamellar liposomes were prepared as follows: 52.5 mg of DPPC, 33.5 mg of cholesterol, 19.9 mg of dicetyl phosphate and 10.0 mg of MPB-PE were placed in a 100 ml round bottom flask and dissolved in 10 ml chloroform and 25 ml tert-butanol. The solvents were evaporated in a rotary evaporator at 55°C, leaving a lipid film on the wall of the flask. Solvent traces were subsequently removed by leaving the flask in a vacuum desiccator overnight

One ml of buffer A (110 mM KCl, 4 mM NaCl, 1 mM $MgCl_2$, 0.1 mM $CaCl_2$, 2 mM MOPS and adjusted to pH 7.4 with 1N KCl) was added to the dry lipid film and vortexed until all the lipids were dispersed (approx. 15 min). The dispersed lipids were then subjected to 10 freeze (-80°C for 30 minutes)/thaw (room temperature water for 10 minutes) cycles and then to 5 cycles of pressurized extrusion (at 500-600 psi) through a 0.4 $\mu$m pore size polycarbonate filter (Nucleopore, Pleasanton, CA). The latter process was carried out in an extruder (Lipex Biomembranes. Inc. Vancouver, and BC, CANADA) maintained at 37°C. The resultant liposome solution was stored in the refrigerator until further use.

Modification of antibody by the heterobifunctional reagent SPDP

The reaction scheme for the conjugation process is shown in Figure 10. 1.6 $\mu$l of 20 mM SPDP was added to 1 ml of PBS buffer containing 1 mg of antibody in a glass tube, resulting in a 5:1 molar ratio of SPDP:antibody. This reaction mixture was incubated for overnight at room temperature (21°C). Unreacted SPDP was separated from the antibody by column chromatography using a Sephadex G-25 column equilibrated with 100 mM sodium acetate buffer (pH 4.5) acetate buffer (pH 4.5). Fractions were monitored by measuring the optical density at 280 nm. The SPDP was reduced to the thiol form by addition of DTT to a final concentration of 50 mM. After 20 minutes at room temperature the modified antibody was separated from the excess DTT by passing through a Sephadex G-25 column pre-equilibrated with 10 mM HEPES buffered saline, pH 8.0.

Conjugation of modified antibody to liposomes

100 $\mu$l of the antibody modified with SPDP was mixed with 100 $\mu$l of the liposome solution and incubated at room temperature for 24 hours with constant stirring under a nitrogen stream.

Separation of the conjugated liposomes from the noncoupled antibody

200 $\mu$l of the conjugated liposome solution was aliquoted into a 10 ml centrifuge tube and 1.5 ml of 40% (w/v) metrizamide was layered over the liposome mixture. This was followed by layers of 2.5 ml 20% (w/v) metrizamide and 0.5 ml PBS. This gradient was centrifuged for 30 minutes at 34,000 rpm (143,000 g). On centrifugation, the liposomes migrate out of the lower layer to the interface between the second medium layer and the buffer layer on the top of the gradient. The unbound antibody remains at the bottom of the gradient. The fraction containing the antibody-liposome conjugates was collected, diluted with the buffer A to a phospholipid concentration of 1 mg/ml and stored in the refrigerator until use as the spreading solution for monolayer preparation.

Monolayer formation and deposition

Monolayers were prepared and transferred to sensor substrates as described in Example 1 for the following solutions:

(a) 80 $\mu$l of the liposome-antibody conjugate spreading solution described above; and
(b) 80 $\mu$l of the same liposome solution without antibody. Both monolayers were deposited as Y type films with a transfer ratio of 1 ($\pm$0.1).

**Example 5**

Detection of the Presence of Pork Protein

Both the above sensors were tested with a pork solution as described in testing of bacterial sensors. To extract meat protein the following procedures were used. Raw or cooked (100°C, 20 min) lean pork meat was ground, and extracted by adding 20 ml of saline solution to 10g of meat and homogenized. The slurry was held at 4°C for 2 hours and then filtered through Whatman No.1 filter paper. The presence of pork antigen was detected in less than 1 minute, and a steady state signal was reached after about 3 minutes (Figure 11).

**Claims**

1. A method for forming a monolayer, comprising:

   (a) providing a composition comprising at least one amphiphilic compound, wherein said composition contains not more than 25% of a volatile organic solvent and may optionally contain one or more compounds of interest;
   (b) immersing one end of a wettable planar surface into an aqueous subphase, wherein said planar surface forms an angle of about 90-170 degrees to an upper surface of said subphase, and said subphase comprises at least one monovalent cation and at least one bivalent cation;
   (c) delivering said composition at a rate of about 0.02-4.0 ml per minute to said planar surface to form a monolayer; and
   (d) compressing said monolayer to a desired surface pressure.

2. The method of claim 1, wherein said rate is about 0.05 - 0.75 ml per minute and said angle is about 140-170 degrees.

3. The method of claim 2, wherein said rate is about 0.1 ml per minute and said angle is about 165 degrees.

4. The method of claim 1, wherein said planar surface is glass.

5. The method of claim 1, wherein said compound of interest is specifically reactive with a microorganism; virus, parasite, toxin, foodborne pathogen, meat or a component thereof.

6. The method of claim 1, wherein said composition is a blood serum.

7. The method of claim 6, wherein said blood serum comprises an antiserum that is specifically reactive with at least one antigen or epitope.

8. The method of claim 7, wherein said antigen or epitope is a microorganism or a component thereof.

9. The method of claim 8, wherein said microorganism is a gram-positive or gram-negative bacterium.

10. The method of claim 9, wherein said microorganism is *Escherichia coli, Salmonella, Campylobacter jejuni, Listeria monocytogenes, Staphylococcus aureus* or *Clostridium perfringens.*

11. The method of claim 1, wherein said amphiphilic compound is a lipid.

12. The method of claim 1, wherein said amphiphilic compound is in a liposome form.

13. The method of claim 12, wherein said compound of interest is conjugated to said liposome.

14. The method of claim 13, wherein said compound of interest is an antibody, protein, polypeptide, nucleic acid, carbohydrate or derivative thereof.

15. The method of claim 14, wherein said antibody or derivative thereof is specifically reactive with at least one antigen from a microorganism, virus, parasite, toxin, foodborne pathogen, raw meat, cooked meat or thermostable meat polypeptide.

16. The method of claim 15, wherein said antigen is from raw or cooked pork chicken, turkey, duck, fish, seafood, shellfish, fruits, vegetables, juice, or dairy products.

17. The method of claim 16, wherein said antibody has the specificity of monoclonal antibody 5H9.

**18.** A method of claim 1, further comprising depositing at least one such monolayer onto a substrate to form an LB film.

**19.** The method of claim 18, wherein said substrate comprises a piezoelectric crystal.

**Patentansprüche**

**1.** Verfahren zum Bilden einer Monolayer, umfassend:

(a) Bereitstellen einer Zusammensetzung, umfassend mindestens eine amphiphile Verbindung, wobei die Zusammensetzung nicht mehr als 25 % eines flüchtigen organischen Lösungsmittels enthält und optional eine oder mehrere Verbindungen von Interesse enthalten kann;
(b) Eintauchen eines Endes einer benetzbaren planaren Oberfläche in eine wässrige Subphase, wobei die planare Oberfläche einen Winkel von etwa 90-170 Grad zu einer oberen Oberfläche der Subphase bildet und die Subphase mindestens ein einwertiges Kation und mindestens ein zweiwertiges Kation umfasst;
(c) Abgeben der Zusammensetzung mit einer Geschwindigkeit von etwa 0,02-4,0 ml pro Minute an die planare Oberfläche, um eine Monolayer zu bilden; und
(d) Komprimieren der Monolayer bis zu einem angestrebten Oberflächendruck.

**2.** Verfahren nach Anspruch 1, wobei die Geschwindigkeit etwa 0,05-0,75 ml pro Minute ist und der Winkel etwa 140-170 Grad ist.

**3.** Verfahren nach Anspruch 2, wobei die Geschwindigkeit etwa 0,1 ml pro Minute ist und der Winkel etwa 165 Grad ist.

**4.** Verfahren nach Anspruch 1, wobei die planare Oberfläche Glas ist.

**5.** Verfahren nach Anspruch 1, wobei die Verbindung von Interesse spezifisch reaktiv mit einem Mikroorganismus, Virus, Parasiten, Toxin, Lebensmittel-übertragenen Pathogen, Fleisch oder einer Komponente davon ist.

**6.** Verfahren nach Anspruch 1, wobei die Zusammensetzung ein Blutserum ist.

**7.** Verfahren nach Anspruch 6, wobei das Blutserum ein Antiserum umfasst, das spezifisch reaktiv für mindestens ein Antigen oder Epitop ist.

**8.** Verfahren nach Anspruch 7, wobei das Antigen oder Epitop ein Mikroorganismus oder eine Komponente davon ist.

**9.** Verfahren nach Anspruch 8, wobei der Mikroorganismus ein gram-positives oder gram-negatives Bakterium ist.

**10.** Verfahren nach Anspruch 9, wobei der Mikroorganismus *Escherichia coli, Salmonella, Campylobacter jejuni, Listeria monocytogenes, Staphylococcus aureus* oder *Clostridium perfringens* ist.

**11.** Verfahren nach Anspruch 1, wobei die amphiphile Verbindung ein Lipid ist.

**12.** Verfahren nach Anspruch 1, wobei die amphiphile Verbindung in der Form eines Liposoms ist.

**13.** Verfahren nach Anspruch 12, wobei die Verbindung von Interesse mit dem Liposom konjugiert ist.

**14.** Verfahren nach Anspruch 13, wobei die Verbindung von Interesse ein Antikörper, Protein, Polypeptid, eine Nukleinsäure, ein Kohlenhydrat oder Derivat davon ist.

**15.** Verfahren nach Anspruch 14, wobei der Antikörper oder ein Derivat davon spezifisch reaktiv mit mindestens einem Antigen aus einem Mikroorganismus, Virus, Parasiten, Toxin, Lebensmittel-übertragenen Pathogen, aus rohem Fleisch, gekochtem Fleisch oder einem thermostabilen Fleischpolypeptid ist.

**16.** Verfahren nach Anspruch 15, wobei das Antigen aus rohem oder gekochtem Schwein, Huhn, Truthahn, Ente, Fisch, Meeresfrüchten, Schalentieren, Früchten, Gemüsen, Saft oder Milchprodukten stammt .

**17.** Verfahren nach Anspruch 16, wobei der Antikörper die Spezifität des monoklonalen Antikörpers 5H9 hat.

**18.** Verfahren nach Anspruch 1, weiterhin umfassend das Ablagern von mindestens einer solchen Monolayer auf einem Substrat, um einen LB-Film zu bilden.

**19.** Verfahren nach Anspruch 18, wobei das Substrat einen piezoelektrischen Kristall umfasst.

**Revendications**

**1.** Méthode pour former une monocouche, comprenant :

(a) la fourniture d'une composition comprenant au moins un composé amphiphile, dans laquelle ladite composition ne contient pas plus de 25 % d'un solvant organique volatil et peut éventuellement contenir un ou plusieurs composés d'intérêt ;
(b) l'immersion d'une extrémité d'une surface plane mouillable dans une sous-phase aqueuse, dans laquelle ladite surface plane forme un angle d'environ 90 à 170 degrés avec une surface supérieure de ladite sous-phase, et ladite sous-phase comprend au moins un cation monovalent et au moins un cation bivalent ;
(c) la fourniture de ladite composition à une vitesse d'environ 0,02 à 4,0 ml par minute à ladite surface plane pour former une monocouche ; et
(d) la compression de ladite monocouche à une pression de surface souhaitée.

**2.** Méthode de la revendication 1, dans laquelle ladite vitesse est d'environ 0,05 à 0,75 ml par minute et ledit angle est d'environ 140 à 170 degrés.

**3.** Méthode de la revendication 2, dans laquelle ladite vitesse est d'environ 0,1 ml par minute et ledit angle est d'environ 165 degrés.

**4.** Méthode de la revendication 1, dans laquelle ladite surface plane est du verre.

**5.** Méthode de la revendication 1, dans laquelle ledit composé d'intérêt réagit spécifiquement avec un micro-organisme, virus, parasite, toxine, pathogène alimentaire, viande ou un composant de ceux-ci.

**6.** Méthode de la revendication 1, dans laquelle ladite composition est un sérum sanguin.

**7.** Méthode de la revendication 6, dans laquelle ledit sérum sanguin comprend un antisérum qui réagit spécifiquement avec au moins un antigène ou un épitope.

**8.** Méthode de la revendication 7, dans laquelle ledit antigène ou épitope est un micro-organisme ou un composant de celui-ci.

**9.** Méthode de la revendication 8, dans laquelle ledit micro-organisme est une bactérie à Gram positif ou à Gram négatif.

**10.** Méthode de la revendication 9, dans laquelle ledit micro-organisme est *Escherichia coli, Salmonella, Campylobacter jejuni, Listeria monocytogenes, Staphylococcus aureus* ou *Clostridium perfringens.*

**11.** Méthode de la revendication 1, dans laquelle ledit composé amphiphile est un lipide.

**12.** Méthode de la revendication 1, dans laquelle ledit composé amphiphile est sous la forme de liposome.

**13.** Méthode de la revendication 12, dans laquelle ledit composé d'intérêt est conjugué audit liposome.

**14.** Méthode de la revendication 13, dans laquelle ledit composé d'intérêt est un anticorps, une protéine, un polypeptide, un acide nucléique, un glucide ou un dérivé de ceux-ci.

**15.** Méthode de la revendication 14, dans laquelle ledit anticorps ou dérivé de celui-ci réagit spécifiquement avec au moins un antigène d'un micro-organisme, virus, parasite, toxine, pathogène alimentaire, viande crue, viande cuite ou polypeptide de viande thermostable.

**16.** Méthode de la revendication 15, dans laquelle ledit antigène provient de porc, poulet, dinde, canard, poisson, fruit de mer, crustacé, fruits, légumes, jus ou produits laitiers, crus ou cuits.

**17.** Méthode de la revendication 16, dans laquelle ledit anticorps a la spécificité d'un anticorps monoclonal 5H9.

**18.** Méthode de la revendication 1, comprenant en outre le dépôt d'au moins une telle monocouche sur un substrat pour former un film LB.

**19.** Méthode de la revendication 18, dans laquelle ledit substrat comprend un cristal piézoélectrique.

FIG. 1.

FIG. 2A.

FIG. 2B.

FIG.3.

FIG. 4.

FIG. 5.

SALMONELLA DETECTION

VOLTAGE, V

—CRYSTAL #3, SUBPHASE A
·······SALMONELLA ($10^4$) BACTERIA/ML
········SALMONELLA ($10^5$) BACTERIA/ML
·-·-·SALMONELLA ($10^6$) BACTERIA/ML
—SALMONELLA ($10^7$) BACTERIA/ML
—SALMONELLA ($10^8$) BACTERIA/ML
----SALMONELLA ($10^9$) BACTERIA/ML

TIME, SEC

FIG. 6A.

SALMONELLA DETECTION

$\Delta V$, V

SALMONELLA, LOG (C)

FIG. 6B.

EP 1 186 059 B1

DETECTION LIMIT

DOSE RESPONSE

$[S]_{lim} \sim 200$ CELLS/ML

SALMONELLA, CELLS/ML

FIG. 7A.

DETECTION LIMIT

HILL PLOT

$K_d = 230$ CELLS/ML

SALMONELLA, CELLS/ML

FIG. 7B.

EP 1 186 059 B1

FIG. 8.

**SENSOR LONGEVITY**

**32 DAYS**

VOLTAGE (V) vs TIME(S)

— SUBPHASE
······ 1/625 SALMONELLA
- - 1/125 SALMONELLA
— 1/25 SALMONELLA
-·- 1/5 SALMONELLA
— SALMONELLA CONCENTRATE

**FIG. 9A.**

**SENSOR LONGEVITY**

**32 DAYS**

VOLTAGE (V) vs LOG[SALMONELLA]

**FIG. 9B.**

EP 1 186 059 B1

SLOPE OF THE ΔVVs LOG [SALMONELLA] GRAPH
AS A FUNCTION OF THE AGE OF THE SENSOR.
(SENSORS WERE STORED AT 5°C)

FIG. 9C.

SLOPE OF THE ΔVVs LOG [SALMONELLA] GRAPH
AS A FUNCTION OF THE AGE OF THE SENSOR.
(SENSORS WERE STORED AT 25°C)

FIG. 9D.

EP 1 186 059 B1

FIG. 10.

SENSOR CONTAINING NO MAB

SENSOR CONTAINING MAB

SOLUTION WITH THE TESTED PROTEIN
IS APPLIED TO THE SENSOR

VOLTAGE, V

5.0

4.5

4.0

3.5

3.0

2.5

2.0

05:57:16pm          06:00:36pm

TIME, HR,MIN,SEC

FIG. 11.

EP 1 186 059 B1